(19) Europäisches Patentamt
European Patent Office
Office européen des brevets

(11) Numéro de publication : **0 057 631**
**B1**

# (12) FASCICULE DE BREVET EUROPÉEN

(45) Date de publication du fascicule du brevet : **20.02.85**

(51) Int. Cl.⁴ : **C 07 D209/20**

(21) Numéro de dépôt : **82400126.7**

(22) Date de dépôt : **22.01.82**

(54) **Nouveaux dérivés de l'acide ((indol-3 yl)-3 amino-2 propionyloxy)-2 acétique, procédés pour leur préparation et médicaments les contenant.**

(30) Priorité : **02.02.81 FR 8101931**

(43) Date de publication de la demande : **11.08.82 Bulletin 82/32**

(45) Mention de la délivrance du brevet : **20.02.85 Bulletin 85/08**

(84) Etats contractants désignés : **AT BE CH DE GB IT LI LU NL SE**

(56) Documents cités : **FR-A- 2 138 046**

(73) Titulaire : **PANMEDICA**
**Zone Industrielle - Ilot J**
**F-06510 Carros (FR)**

(72) Inventeur : **Laruelle, Claude Octave**
**Avenue Bellevue**
**F-06270 Villeneuve Loubet (FR)**
Inventeur : **Lepant, Marcel**
**l'Escoundu Allée Clairefontaine**
**F-06140 Vence (FR)**

(74) Mandataire : **Ores, Irène et al**
**CABINET ORES 6, Avenue de Messine**
**F-75008 Paris (FR)**

**EP 0 057 631 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention est relative à de nouveaux dérivés de l'acide [(indol-3 yl)-3 amino-2 propionyloxy]-2 acétique, ainsi qu'à des procédés pour leur préparation et médicaments les contenant.

On connaît le rôle que joue la sérotonine (ou hydroxy-5 tryptamine) dans l'activité biologique des êtres vivants. Outre qu'elle régularise la motilité intestinale, qu'elle agit sur la musculature lisse, outre ses effets vasoconstricteurs, elle exerce également des effets psychomoteurs et psychomimétiques, constituant ainsi une véritable neurohormone qui tient une place considérable comme médiateur chimique dans l'activité cérébrale.

La déficience du système sérotoninergique (la sérotonine est principalement sécrétée par les cellules argentaffines, en particulier au niveau du tube digestif et surtout au niveau de l'encéphale) peut provoquer des troubles psychiatriques et neurologiques très graves. Comme il est impossible de fournir de la sérotonine préformée aux systèmes sérotoninergiques insuffisants, son précurseur immédiat à savoir le 5-hydroxy-tryptophane (5 HTP) dont la formule figure ci-dessous, était encore jusqu'à présent le meilleur vecteur assimilable de sérotonine.

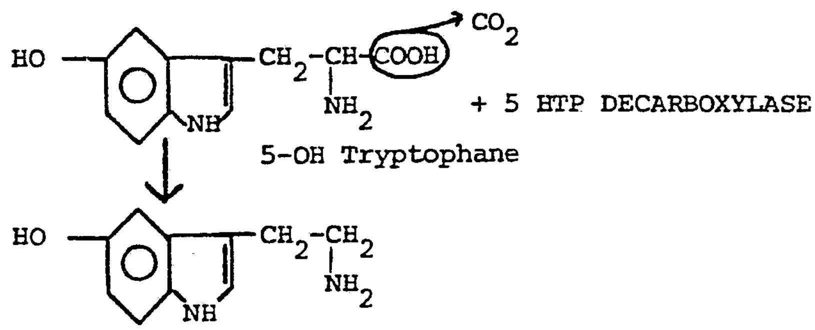

5-OH Tryptophane

5-OH tryptamine
ou Sérotonine

Une telle administration constitue cependant un inconvénient important : la décarboxylase transformant le 5-hydroxytryptophane en sérotonine étant largement répandue dans le système périphérique, elle entraîne une perte considérable de 5-HTP avant son passage cérébral.

La présente invention s'est en conséquence donné pour but de pourvoir à de nouveaux dérivés de 5-HTP plus résistants à la décarboxylation périphérique et régénérant le 5-HTP au niveau cérébral, augmentant ainsi le bilan de passage de la barrière hémato-encéphalique. Le but de la présente invention est donc de permettre — sans requérir l'introduction d'un inhibiteur de la décarboxylase périphérique — d'augmenter le taux de sérotonine endogène dans le cerveau en réduisant considérablement la déperdition périphérique de 5-HTP.

La présente invention a pour objet des dérivés de l'acide [(indol-3 yl)-3 amino-2 propionyloxy]-2 acétique caractérisés en ce qu'ils répondent à la formule générale I ci-après :

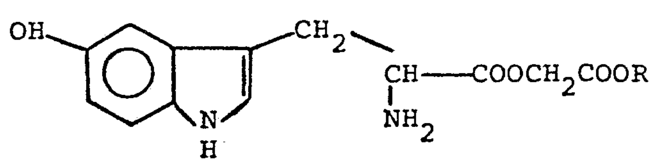
(I)

dans laquelle R représente :

— un atome d'hydrogène,

— un radical alkyle linéaire ou ramifié renfermant 1 à 12 atomes de carbone,

— ou un radical alicyclique mono- ou polycyclique renfermant 5 à 16 atomes de carbone éventuellement lié par un radical méthylène

ainsi que leurs sels d'addition pharmaceutiquement acceptables.

Les composés conformes à la présente invention sont de remarquables médicaments utilisables en thérapeutique humaine et vétérinaire et notamment dans le domaine psychiatrique.

La présente invention est également relative à un procédé de préparation de dérivés conformes à la présente invention, caractérisé en ce que l'on bloque la fonction phénol et/ou la fonction amine par un radical $R_2OCO$— où $R_2$ représente un groupe aliphatique ou aromatique destiné à protéger la fonction phénol et/ou amine, en ce que l'on estérifie l'acide de départ par un composé répondant à la formule

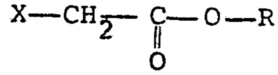

dans laquelle :

X représente un atome d'halogène et de préférence un atome de chlore ou de brome, et

R a la même signification que ci-dessus,

et en ce que l'on débloque ensuite la fonction amine et/ou la fonction phénol.

Selon un mode de réalisation avantageux du procédé objet de la présente invention, les fonctions amine et/ou phénol sont bloquées à l'aide de réactifs pris dans le groupe qui comprend l'

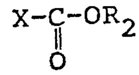

et

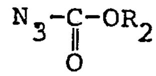

$R_2$ et X ayant la même signification que précédemment.

Le schéma réactionnel peut être représenté de la façon suivante :

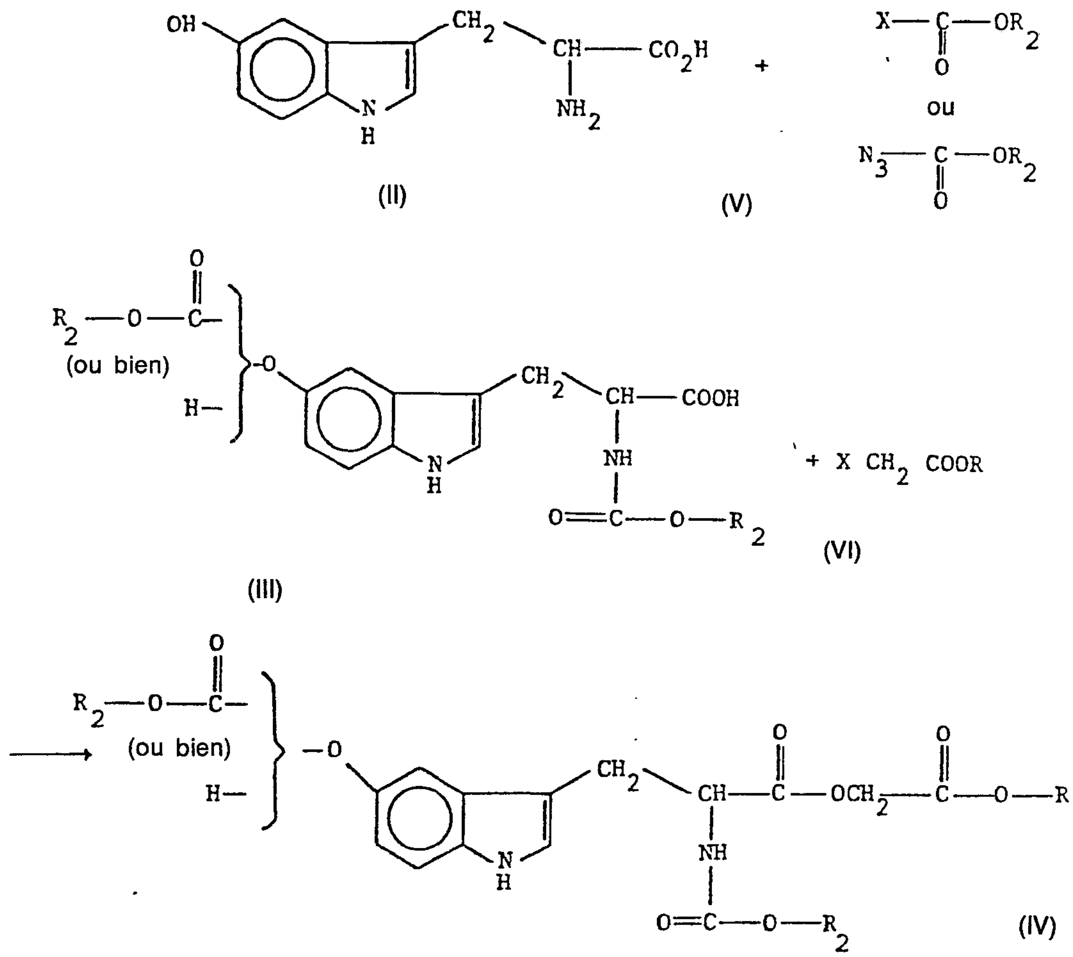

(II) (V) (VI) (III) (IV)

Puis par déblocage de l'OH en 5 et/ou du $NH_2$

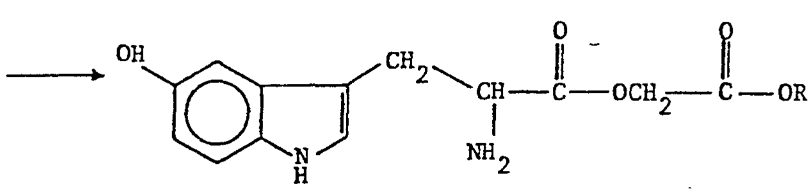

(VII)

Suivant un autre mode de réalisation avantageux de l'objet de l'invention, la condensation des dérivés de l'acide (hydroxy-5 indol-3 yl)-3 aminopropionique avec l'agent de blocage

$$X-\underset{\underset{O}{\|}}{C}-OR_2$$

s'effectue en présence d'une base acceptrice d'acide halohydrique et dans un solvant aprotique tel que le diméthylformamide ou analogue.

Conformément à l'invention, le déblocage des fonctions $NH_2$ et/ou OH est réalisé par un traitement acide.

Selon une autre modalité du procédé conforme à la présente invention, le déblocage des fonctions $NH_2$ et/ou OH est réalisé par hydrogénation catalytique.

Les réactions chimiques pratiquées ne modifient pas la stéréochimie au niveau du carbone asymétrique porteur de l'azote amine primaire. On ne note pas non plus de racémisation des dérivés optiquement actifs.

Outre les dispositions qui précèdent, l'invention comprend encore d'autres dispositions, qui ressortiront de la description qui va suivre.

La présente invention pourra être mieux comprise à l'aide du complément de description qui va suivre, dans lequel on trouvera des exemples de mise en œuvre du procédé de préparation des nouveaux dérivés conformes à l'invention, ainsi qu'un compte-rendu d'expérimentations pharmacologiques.

Il doit être bien entendu toutefois que ces exemples sont donnés uniquement à titre d'illustration de l'objet de l'invention, mais n'en constituent en aucune manière une limitation.

Il est à noter que les spectres RMN$^1$H des produits de formule I présentent tous les signaux caractéristiques communs suivants :

6,6 à 7,6 ppm (m) (aromatiques) (4H) ; 4,7 ppm (s)$—O—CH_2—C{=}O$ ;

3,8 ppm (m) x $NH_2—\underline{CH}—COO$ (1H) ; 3,1 ppm (m) $\underline{CH_2}—CH(NH_2)—COO$.

Les spectres IR (K Br) présentent les bandes caractéristiques communes suivantes :

3 300 $cm^{-1}$ (OH, $NH_2$), 1 745-1 755 $cm^{-1}$ (esters).

## Exemple 1

D,L-[hydroxy-5 indol-3 yl)-3 amino-2 propionyloxy]-2 acétate d'éthyle : [(I) ; $R = C_2H_5$]

a) Acide D,L-(benzyloxycarbonyloxy-5 indol-3 yl)-3 benzyloxycarbonylamino-2 propionique

On dissout sous atmosphère d'azote 220 g de DL hydroxy-5 tryptophane (1 mole) dans 5 litres d'eau et 40 g de soude (1 mole). On coule en quatre heures sur cette solution agitée 660 ml de chloroformiate de benzyle en solution à 50 % dans le toluène tout en maintenant le pH vers 9,5 par addition de soude 2 N. Après quelques heures d'agitation à température ordinaire, on décante la phase aqueuse, la lave à l'éther, puis coule sur un excès d'acide chlorhydrique dilué glacé, le précipité est filtré, lavé, séché. Ce produit brut est purifié dans un mélange propanol-eau puis recristallisé dans 20 volumes d'éthanol.

On obtient un premier jet de 133 g de cristaux blancs de pF 135/140 °C (Kofler) (rendement = 31 %) et un deuxième jet de 200 g de point de fusion 110/120 °C (rendement total = 72 %) utilisable pour la suite des synthèses.

b) D,L-[(benzyloxycarbonyloxy-5 indol-3 yl)-3 benzyloxycarbonylamino-2 propionyloxy]-2 acétate d'éthyle

On mélange :

— 100 g (0,2 mole) d'acide D,L-(benzyloxycarbonyloxy-5 indol-3 yl)-3 benzyloxycarbonylamino-2 propionique (préparé selon exemple 1-a),

— 600 ml de diméthylformamide,

— 40 ml (0,2 mole) de dicyclohexylamine,

— 21,1 ml (0,2 mole) de chloracétate d'éthyle,

— 1 g d'iodure de sodium.

Après 20 heures d'agitation à température ordinaire, on filtre le précipité de chlorhydrate de dicyclohexylamine, le lave à l'acétate d'éthyle et évapore les solvants sous pression réduite. Le résidu huileux est repris dans 1 litre de toluène et lavé successivement à l'eau, à l'acide chlorhydrique dilué, puis au bicarbonate de sodium dilué. Après séchage, le solvant est évaporé et l'huile résiduelle est cristallisée dans l'hexane puis recristallisée dans le xylène. On obtient un produit cristallisé blanc de pF = 74 °C (Kofler).

c) D,L-[(hydroxy-5 indol-3 yl)-3 amino-2 propionyloxy]-2 acétate d'éthyle

On hydrogène 25 g (0,043 mole) du dérivé précédent dans 500 ml d'alcool isopropylique en présence de 2,5 g de Palladium à 5 % sur charbon. Quand la théorie d'hydrogène est absorbée, on filtre le catalyseur, évapore, reprend à l'acétate d'éthyle et lave à l'eau. Après évaporation du solvant, le résidu huileux est recristallisé dans le cyclohexane. On obtient 60 % de produit pur fondant à 93/94 °C.

d) Acide D,L-(hydroxy-5 indol-3 yl)-3 benzyloxycarbonylamino-2 propionique : [(III), OH-5 et R' = benzyl]

On dissout à température ordinaire sous atmosphère d'azote 44 g (0,2 mole) de DL-hydroxy-5 tryptophane dans 1 litre d'eau distillée et 6 g de soude pure, puis coule en 2 heures 28,5 ml de chloroformiate de benzyle en solution dans 150 ml d'acétone en maintenant le pH de la solution à 9,5 par ajout de soude diluée. Après quelques heures d'agitation à température ordinaire, on évapore l'acétone sous pression réduite et coule la solution aqueuse alcaline sur un excès d'acide chlorhydrique dilué glacé. Le précipité est filtré, lavé rapidement et séché à l'abri de l'air et de la lumière. Le produit brut ($R^{dt}$ 100 %) est purifié par chromatographie sur silice à l'abri de la lumière par élution avec un mélange benzène méthanol on obtient 26 g ($R^{dt}$ = 37 %) de produit pur de pF = 80 °C.

e) D,L-[(hydroxy-5 indol-3 yl)-3 benzyloxycarbonylamino-2 propionyloxy]-2 acétate d'éthyle

On agite pendant 20 heures à température ordinaire le mélange suivant :

— 17,7 g (0,05 mole) d'acide DL (hydroxy-5 indol-3 yl)-3 benzyloxycarbonylamino-2 propionique préparé selon l'exemple 1-d,
— 0,5 g d'iodure de sodium,
— 6,1 g (0,05 mole) de chloracétate d'éthyle,
— 10 ml de dicyclohexylamine,
— 100 ml de diméthylformamide.

On traite ensuite selon l'exemple 1-b. L'huile brute résiduelle est chromatographiée sur silice en éluant par un mélange benzène/acétate d'éthyle. On obtient 16,2 g d'huile épaisse ne présentant qu'un seul spot de Rf 0,55 par C.C.M. sur gel de silice dans le système solvant : toluène 80, formiate d'éthyle 40, acide formique 5.

Le spectre de R.M.N. $^1H$ enregistré en solution dans DMSO $D^6$ présente les signaux caractéristiques suivants : 7,3 ppm (s) (5H) ar. du benzyle ; 6,8/7,2 ppm (m) (4H) ar. de l'indole ; 5,2 ppm (s) (2H) $CH_2$ du benzyle ; 4,7 ppm (s) (2H) $-O-CH_2-\overset{|}{C}=O$ ; 4,2 ppm (m) (2H) de $CH_2-CH_3$ ; 4,2 ppm (m) (1H) du CH en α de $NH-\overset{O}{\overset{\|}{C}}-O$ ; 3,3 ppm (m) (2H) $\underline{CH_2}-CH$ ; 1,2 ppm $CH_3$ (t) (3H).

f) D,L-[(hydroxy-5 indol-3 yl)-3 amino-2 propionyloxy]-2 acétate d'éthyle

On hydrogène 8,8 g (0,020 mole) du produit précédemment obtenu en solution dans 150 ml d'alcool isopropylique en présence de 1 g de Palladium à 5 % sur charbon. On traite ensuite selon l'exemple 1-c et l'on obtient le composé du titre de pF 93/94 °C identique à celui préparé selon la séquence 1-a, 1-b, 1-c.

## Exemple 2

D,L-[(hydroxy-5 indol-3 yl)-3 amino propionyloxy]-2 acétate de butyle : [(I), R = $C_4H_9$]

a) D,L-[(benzyloxycarbonyloxy-5 indol-3 yl)-3 benzyloxycarbonylamino-2 propionyloxy]-2 acétate de butyle

On mélange à température ordinaire :

— 100 g (0,2 mole) d'acide D,L (benzyloxycarbonyloxy-5 indol-3 yl)-3 benzyloxycarbonylamino-2 propionique (préparé selon l'exemple 1-a),
— 40 ml (0,2 mole) de dicyclohexylamine,
— 28,3 ml (0,2 mole) de chloracétate de butyle,
— 600 ml de diméthylformamide,
— 2 g d'iodure de sodium,

puis traite selon la technique décrite à l'exemple 1-b.

On obtient 125 g de produit brut qui peut être purifié par cristallisation dans l'isopropanol puis dans l'éthanol. On obtient ainsi un produit de pF 90 °C (Kofler).

b) D,L-[(hydroxy-5 indol-3 yl)-3 amino-2 propionyloxy]-2 acétate de butyle

On hydrogène 25 g de produit précédemment obtenu dans 500 ml d'alcool isopropylique en

présence de 2,5 g de Palladium à 5 % sur charbon. Quand la théorie d'hydrogène a été absorbée, on filtre le catalyseur, évapore puis reprend à l'acétate d'éthyle. Après lavage à l'eau, le solvant est évaporé. Le produit est purifié par chromatographie sur silice, on obtient 50 % de produit fondant à 90 °C.

## Exemple 3

D,L-[(hydroxy-5 indol-3 yl)-3 amino-2 propionyloxy]-2 acétate d'octyle : [(I), R = $C_8H_{17}$]

a) D,L-[(benzyloxycarbonyloxy-5 indol-3 yl)-3 benzyloxycarbonylamino-2 propionyloxy]-2 acétate d'octyle

On agite pendant 20 heures le mélange suivant :
— 100 g (0,2 mole) d'acide D,L-(benzyloxycarbonyloxy-5 indol-3 yl)-3 benzyloxycarbonylamino-2 propionique,
— 600 ml de diméthylformamide,
— 40 ml (0,2 mole) de dicyclohexylamine,
— 2 g d'iodure de sodium,
— 41,3 g (0,2 mole) de chloracétate d'octyle,
puis traite suivant la technique décrite à l'exemple 1-b. Le produit brut, recristallisé dans le xylène donne 36 g de beaux cristaux blancs de pF 96 °C.

b) D,L-[(hydroxy-5 indol-3 yl)-3 amino-2 propionyloxy]-2 acétate d'octyle

On hydrogène à pression atmosphérique 50 g (0,075 mole) du composé précédemment obtenu dans 500 ml d'alcool isopropylique en présence de 5 g de Palladium à 5 % sur charbon. Quand la théorie d'hydrogène a été consommée, on filtre le catalyseur, évapore à sec et reprend à l'acétate d'éthyle. La couche organique est lavée à l'eau, traitée au noir puis évaporée. On obtient une huile brute qu'on cristallise dans le cyclohexane. pF = 91 °C.

## Exemple 4

D,L-[(hydroxy-5 indol-3 yl)-3 amino-2 propionyloxy]-2 acétate de dodécyle : [(I), R = $C_{12}H_{25}$]

a) D,L-[(benzyloxycarbonyloxy-5 indol-3 yl)-3 benzyloxycarbonylamino-2 propionyloxy]-2 acétate de dodécyle

On mélange :
— 25 g (0,05 mole) d'acide D,L-(benzyloxycarbonyloxy-5 indol-3 yl)-3 benzyloxycarbonylamino-2 propionique (préparé selon l'exemple 1-a),
— 200 ml de diméthylformamide,
— 10 ml de dicyclohexylamine,
— 500 mg d'iodure de sodium,
— 13,2 g de chloracétate de dodécyle,
puis traite suivant la technique décrite à l'exemple 1-b. Le produit peut être recristallisé dans un mélange isopropanol hexane.

b) D,L-[(hydroxy-5 indol-3 yl)-3 amino-2 propionyloxy]-2 acétate de dodécyle

On hydrogène 25 g du produit obtenu précédemment dans 500 ml d'alcool isopropylique en présence de 2,5 g de Palladium à 5 % sur charbon.

Quand la théorie d'hydrogène a été absorbée, on filtre le catalyseur, évapore à sec, reprend par de l'acétate d'éthyle qu'on lave à l'eau. Par traitement avec de l'acide chlorhydrique, on peut isoler directement le chlorhydrate pF = 122/4 °C.

## Exemple 5

L-[(hydroxy-5 indol-3 yl)-3 amino-2 propionyloxy]-2 acétate de méthyle : [(I), R = $CH_3$]

a) Acide L-(benzyloxycarbonyloxy-5 indol-3 yl) benzyloxycarbonylamino-2 propionique

On dissout sous atmosphère d'azote 220 g de (L) hydroxy-5 tryptophane dans 5 litres d'eau et traite au chloroformiate de benzyle selon le procédé employé à l'exemple 1-a pour le racémique. On obtient ainsi 93 % d'huile brute. On purifie le produit brut selon la technique employée pour le D-L. On obtient 40 % de cristaux blancs fondant à 145/6 °C.
$[\alpha]^{21}_D = -7,1°$ (C = 0,5 % éthanol absolu)
$[\alpha]^{21}_D = -9,9°$ (C = 1 % acétone).

b) L-[(benzyloxycarbonyloxy-5 indol-3 yl)-3 benzyloxycarbonylamino-2 propionyloxy]-2 acétate de méthyle

On agite pendant 20 heures le mélange suivant :

— 50 g (0,1 mole) d'acide L-(benzyloxycarbonyloxy-5 indol-3 yl)-3 benzyloxycarbonylamino-2 propionique (préparé selon 5-a),

— 300 ml de diméthyl formamide,

— 20 ml de dicyclohexylamine,

— 1 g d'iodure de sodium,

— 11 g (0,1 mole) de chloracétate de méthyle,

et traite suivant l'exemple 1-b.

Après évaporation du solvant, on purifie par chromatographie sur silice en éluant par un mélange benzène éther. On obtient une huile pure ne présentant qu'un seul spot de Rf 0,6 en C.C.M. sur plaque de silice dans le système toluène 80, formiate d'éthyle 40, acide formique 5 et qui est utilisée directement dans l'étape suivante.

c) L-[(hydroxy-5 indol-3 yl)-3 amino-2 propionyloxy]-2 acétate de méthyle

On hydrogène 28 g (0,05 Mole) du produit précédent en solution dans l'isopropanol et en présence de 2,5 g de Palladium à 5 % sur charbon. Quand la théorie d'hydrogène a été fixée, on filtre le catalyseur, évapore le solvant, reprend dans l'acétate d'éthyle qu'on lave à l'eau, puis après évaporation du solvant recristallise dans le benzène. On obtient 50 % de cristaux blancs fondant à 125 °C (Kofler) $[\alpha]^{25}_D = -59°$ (acétone 1 %).

## Exemple 6

L-[(hydroxy-5 indol-3 yl)-3 amino-2 propionyloxy]-2 acétate d'éthyle : [(I), $R = C_2H_5$]

a) L-[(benzyloxycarbonyloxy-5 indol-3 yl)-3 benzyloxycarbonylamino-2 propionyloxy]-2 acétate d'éthyle

On agite pendant 20 heures le mélange suivant :

— 50 g (0,1 mole) acide L-(benzyloxycarbonyloxy-5 indol-3 yl)-3 benzyloxycarbonylamino-2 propionique obtenu suivant l'exemple 5-a,

— 300 ml de diméthylformamide,

— 20 ml de dicyclohexylamine,

— 1 g d'iodure de sodium,

— 10,5 ml de chloracétate d'éthyle,

et traite selon l'exemple 1-b après évaporation complète du solvant. On obtient 51 g d'une huile brune ne présentant qu'un seul spot de Rf = 0,6 en chromatographie couche mince sur plaque Kieselgel dans le système toluène 80, formiate d'éthyle 40, acide formique 5. Le spectre infrarouge du produit comporte deux bandes fortes caractéristiques à 3 400 $cm^{-1}$ et 1 740 $cm^{-1}$ et un $[\alpha]^{25}_D = -22,55°$ (1 % dans l'éthanol).

b) L-[(hydroxy-5 indol-3 yl)-3 amino-2 propionyloxy]-2 acétate d'éthyle

On hydrogène sous pression atmosphérique 49 g (0,085 4 mole) du produit obtenu précédemment en solution dans l'isopropanol et en présence de 2,5 g de Palladium à 5 % sur charbon. Quand la théorie d'hydrogène a été fixée, on filtre le catalyseur, évapore le solvant et reprend à l'acétate d'éthyle qu'on lave à l'eau, traite au noir puis évapore. Le résidu repris à l'alcool isopropylique est converti en chlorhydrate qui cristallise au froid, filtre, lave et sèche. On obtient 20,8 g (rendement 71 %) de chlorhydrate de pF 257/260°. Ce chlorhydrate traité par de l'ammoniaque dilué, libère la base, qu'on filtre, lave à l'eau et sèche. On obtient de beaux cristaux blancs de pF = 136° ne présentant qu'un seul spot de Rf = 0,4 en C.C.M. sur Kieselgel dans le système acétonitrile 130, eau 15, acide acétique 5-$[\alpha]^{25}_D = -60,4°$ (1 % acétone).

## Exemple 7

L-[(hydroxy-5 indol-3 yl)-3 amino-2 propionyloxy]-2 acétate de n-propyle : [(I), $R = C_3H_7$]

a) L-[(benzyloxycarbonyloxy-5 indol-3 yl)-3 benzyloxycarbonylamino-2 propionyloxy]-2 acétate de propyle

On agite pendant 20 heures le mélange suivant :

— 50 g (0,1 mole) acide L-(benzyloxycarbonyloxy-5 indol-3 yl)-3 benzyloxycarbonylamino-2 propionique obtenu suivant l'exemple 5-a,
— 300 ml de diméthylformamide,
— 20 ml de dicyclohexylamine,
— 1 g d'iodure de sodium,
— 12 ml de chloracétate de n-propyle,
et traite selon l'exemple 1-b. Après évaporation du solvant, on purifie l'huile brute par chromatographie sur silice en éluant par un mélange benzène/éther. On obtient 60 % d'une huile pure ne présentant qu'un seul spot de Rf 0,5 par C.C.M. sur gel de silice dans le système : toluène 80, formiate d'éthyle 40, acide formique 5$[\alpha]^{25}_D = -25°$ (1 % Ethanol).

b) L-[(hydroxy-5 indol-3 yl)-3 amino-2 propionyloxy]-2 acétate de n-propyle

On hydrogène sous pression atmosphérique 29,5 g (0,05 mole) de produit précédemment obtenu dans 500 ml d'alcool isopropylique en présence de 2,5 g de Palladium à 5 % sur charbon. Quand la théorie d'hydrogène a été fixée, on filtre le catalyseur, évapore, reprend à l'acétate d'éthyle puis lave à l'eau. Après évaporation du solvant et recristallisation dans le benzène, on obtient 55 % de produit pur fondant à 97 °C et $[\alpha]^{25}_D = -58,5°$ (acétone 1 %).

## Exemple 8

L-[(hydroxy-5 indol-3 yl)-3 amino-2 propionyloxy]-2 acétate de n-butyle : [(I), R = $C_4H_9$]

a) L-[(benzyloxycarbonyloxy-5 indol-3 yl)-3 benzyloxycarbonylamino-2 propionyloxy]-2 acétate de butyle

On agite pendant 20 heures le mélange suivant :
— 50 g (0,1 mole) d'acide L-(benzyloxycarbonyloxy-5 indol-3 yl) benzyloxy carbonylamino-2 propionique préparé suivant l'exemple 5-a,
— 20 ml de dicyclohexylamine,
— 14,2 ml (0,1 mole) de chloracétate de butyle,
— 300 ml de diméthylformamide,
— 1 g d'iodure de sodium,
puis traite suivant la technique décrite à l'exemple 1-b. On obtient une huile (rendement = 80 %) ne présentant qu'un seul spot en C.C.M. sur gel de silice de Rf = 0,5 dans le système : toluène 80, formiate d'éthyle 40, acide formique 5.

b) L-[(hydroxy-5 indol-3 yl)-3 amino-2 propionyloxy]-2 acétate de n-butyle

On hydrogène sous pression atmosphérique 37,5 g (0,057 mole) de produit précédemment obtenu dans 500 ml d'alcool isopropylique en présence de 2,5 g de Palladium à 5 % sur charbon. Quand la théorie d'hydrogène a été absorbée, on filtre le catalyseur, évapore, reprend à l'acétate d'éthyle et lave à l'eau. Après évaporation du solvant, on obtient 23,5 g d'huile brute, qu'on reprend à l'acétate d'éthyle, lave à l'eau puis évapore. On obtient 51 % de produit pur fondant à 96 °C (Kofler) $[\alpha]^{25}_D = +2,7°$ (Ethanol 1 %) ; $= -51°$ (acétone %).

## Exemple 9

L-[(hydroxy-5 indol-3 yl)-3 amino-2 propionyloxy]-2 acétate de n-pentyle : [(I), R = $C_5H_{11}$]

a) L-[(benzyloxycarbonyloxy-5 indol-3 yl)-3 benzyloxycarbonylamino-2 propionyloxy]-2 acétate de pentyle

On agite pendant 20 heures le mélange suivant :
— 50 g (0,1 mole) d'acide L-(benzyloxycarbonyloxy-5 indol-3 yl)-3 benzyloxycarbonylamino-2 propionique préparé selon l'exemple 5-a,
— 20 ml de dicyclohexylamine (0,1 mole),
— 1 g d'iodure de sodium,
— 16,5 g (0,1 mole) de chloracétate de pentyle,
— 300 ml de diméthylformamide,
puis traite selon l'exemple 1-b. L'huile brute est purifiée par chromatographie sur silice en éluant par un mélange benzène éther. On obtient 62 % d'huile pure ne présentant qu'un seul spot de Rf 0,55 en C.C.M. sur gel de silice dans le système solvant décrit précédemment.

b) L-[(hydroxy-5 indol-3 yl)-3 amino-2 propionyloxy]-2 acétate de n-pentyle

On hydrogène 30 g (0,05 mole) de produit obtenu en a) dans 400 ml d'alcool isopropylique en présence de 2,5 g de Palladium à 5 % sur charbon. Quand la théorie d'hydrogène a été absorbée, on filtre le catalyseur, évapore à sec et reprend par l'acétate d'éthyle qu'on lave à l'eau. Après évaporation du solvant, l'huile brute est cristallisée dans le benzène. On obtient 45 % de produit pur fondant à 97 °C $[\alpha]^{22}_D = -50{,}5°$ (acétone 1 %).

Exemple 10

L-[(hydroxy-5 indol-3 yl)-3 amino-2 propionyloxy]-2 acétate de n-hexyle : [(I), R = $C_6H_{13}$]

a) L-[(benzyloxycarbonyloxy-5 indol-3 yl)-3 benzyloxycarbonylamino-2 propionyloxy]-2 acétate de n-hexyle

On agite pendant 20 heures à température ordinaire le mélange suivant :
— 50 g (0,1 mole) d'acide L-benzyloxycarbonyloxy-5 indol-3 yl)-3 benzyloxycarbonylamino-2 propionique préparé selon l'exemple 5-a,
— 20 ml de dicyclohexylamine (0,1 mole),
— 1 g d'iodure de sodium,
— 18 g (0,1 mole) de chloracétate de n-hexyle,
— 300 ml de diméthylformamide,
puis traite selon l'exemple 1-b. L'huile brute est chromatographiée sur silice par un mélange benzène éther. La fraction pure représente 66 % de rendement, elle ne présente qu'un seul spot en C.C.M. sur gel de silice dans le système solvant habituel, et est utilisée directement pour l'étape suivante.

b) L-[(hydroxy-indol-3 yl) amino-2 propionyloxy]-2 acétate de n-hexyle

On hydrogène sous pression atmosphérique 20 g (0,047 mole) du produit précédemment obtenu en solution dans 500 ml d'alcool isopropylique en présence de 2,5 g de Palladium à 5 % sur charbon. Quand la théorie d'hydrogène a été absorbée, on filtre le catalyseur, évapore l'alcool, reprend à l'acétate d'éthyle qu'on lave soigneusement à l'eau. Après évaporation du solvant, on cristallise dans le benzène et obtient 70 % de cristaux blancs fondant à 99 °C (Kofler) et $[\alpha]^{22}_D = -51°$ (acétone 1 %).

Exemple 11

L-[(hydroxy-5 indol-3 yl)-3 amino-2 propionyloxy]-2 acétate de n-heptyle : [(I), R = $C_7H_{15}$]

a) L-[(benzyloxycarbonyloxy-5 indol-3 yl)-3 benzyloxycarbonylamino-2 propionyloxy]-2 acétate de n-heptyle

On agite pendant 20 heures à température ordinaire :
— 50 g (0,1 mole) d'acide L-(benzyloxycarbonyloxy-5 indol-3 yl)-3 benzyloxycarbonylamino-2 propionique préparé selon l'exemple 5-a,
— 20 ml de dicyclohexylamine (0,1 mole),
— 1 g d'iodure de sodium,
— 19,5 g (0,1 mole) de chloracétate de n-heptyle,
— 300 ml de diméthylformamide,
puis traite suivant l'exemple 1-b. Après purification par chromatographie sur silice avec un mélange benzène éther, on obtient 71 % d'huile brute présentant un seul spot de Rf = 0,55 dans le système solvant habituel et $[\alpha]^{22}_D = -13°$ (Ethanol absolu 1 %).

b) L-[(hydroxy-5 indol-3 yl)-3 amino-2 propionyloxy]-2 acétate de n-heptyle

On hydrogène 32 g (0,05 mole) du produit précédemment obtenu dans 400 ml d'isopropanol en présence de 2,5 g de Palladium à 5 % sur charbon. Quand la théorie d'hydrogène a été fixée, on filtre le catalyseur, évapore le solvant, reprend à l'acétate d'éthyle qu'on lave à l'eau. Après évaporation de ce solvant le résidu brut est recristallisé dans le benzène. On obtient 10 g de cristaux blancs fondant à 99 °C et $[\alpha]^{22}_D = -50{,}7°$ (acétone 1 %).

Exemple 12

L-[(hydroxy-5 indol-3 yl)-3 amino-2 propionyloxy]-2 acétate de n-octyle : [(I), R =$C_8H_{17}$]

a) L-[(benzyloxycarbonyloxy-5 indol-3 yl)-3 benzyloxycarbonylamino-2 propionyloxy]-2 acétate de n-octyle

On agite pendant 20 heures le mélange suivant :

— 50 g (0,1 mole) d'acide (L) (benzyloxycarbonyloxy-5 indol-3 yl)-3 benzyloxycarbonylamino-2 propionique obtenu au paragraphe 5-a,

— 300 ml de diméthylformamide,

— 20 ml de dicyclohexylamine,

— 1 g d'iodure de sodium,

— 20,5 g (0,1 mole) de chloracétate de n-octyle,

et traite suivant l'exemple 1-b.

On obtient 51 g (77 %) d'huile brute présentant un seul spot de Rf = 0,6 en C.C.M. sur gel de silice dans le système solvant habituel $[\alpha]^{25}_D = -13,9°$ (1 % Ethanol).

b) L-[(hydroxy-5 indol-3 yl)-3 amino-2 propionyloxy]-2 acétate de n-octyle

On hydrogène 30 g (0,045 mole) de produit obtenu au paragraphe suivant dans 400 ml d'alcool isopropylique en présence de 2,5 g de Palladium à 5 % sur charbon. Quand la théorie d'hydrogène a été absorbée, on filtre le catalyseur, évapore à sec, reprend par l'acétate d'éthyle qu'on lave à l'eau. Après évaporation du solvant, on recristallise le résidu brut dans le benzène. On obtient 10,9 g de cristaux blancs (rendement 48 %) fondant à 105 °C. $[\alpha]_D = +0,71°$ (EtOH absolu 1 %), $-52,0°$ (acétone absolu 1 %).

## Exemple 13

L-[(hydroxy-5 indol-3 yl)-3 amino-2 propionyloxy]-2 acétate de n-dodécyle : [(I), R = $C_{12}H_{25}$]

a) L-[(benzyloxycarbonyloxy-5 indol-3 yl)-3 benzyloxycarbonylamino-2 propionyloxy]-2 acétate de n-dodécyle

On agite pendant 20 heures le mélange suivant :

— 50 g (0,1 mole) d'acide L-(benzyloxycarbonyloxy-5 indol-3 yl)-3 benzyloxycarbonylamino-2 propionique préparé suivant l'exemple 5-a,

— 20 ml de dicyclohexylamine,

— 1 g d'iodure de sodium,

— 26,4 g de chloracétate de n-dodécyle,

— 300 ml de diméthylformamide,

puis traite suivant l'exemple 1-b. Le produit brut est purifié par chromatographie sur silice. La fraction pure ne présente qu'un seul spot en C.C.M. dans le système solvant habituel (rendement 80 %).

b) L-[(hydroxy-5 indol-3 yl)-3 amino-2 propionyloxy]-2 acétate de n-dodécyle

On hydrogène sous pression atmosphérique 77,4 g du produit précédent en solution dans 1,5 litre d'isopropanol en présence de 7 g de Palladium à 5 % sur charbon. Quand la théorie d'hydrogène a été fixée, on filtre le catalyseur, évapore à sec, reprend à l'acétate d'éthyle et lave à l'eau. Après évaporation du solvant, le résidu brut est cristallisé deux fois dans le benzène. On obtient 60 % de produit pur de pF 107,5 °C $[\alpha]^{25}_D = +0,17$ (1 % Ethanol) $[\alpha]^{25}_D = -42,2°$ (1 % Acétone).

## Exemple 14

L-[(hydroxy-5 indol-3 yl)-3 amino-2 propionyloxy]-2 acétate de (diméthyl-3,3) butyl-2

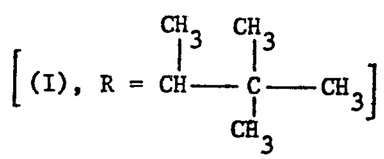

a) Chloracétate de (diméthyl-3,3) butyl-2

On ajoute lentement 47,5 g (0,42 mole) de chlorure de chloracétyle à un mélange de 40,9 g (0,4 mole) de diméthyl-3,3 butanol-2 et de 58 ml (0,42 mole) de triéthylamine. Le mélange réactionnel maintenu à 10 °C pendant la coulée du chlorure d'acide est ramené à température ordinaire et agité pendant environ 4 heures. Après filtration et lavage à l'éther du chlorhydrate de triéthylamine, la solution est lavée à l'eau puis séchée sur sulfate de sodium. Après évaporation du solvant et distillation sous vide, on obtient 64 g soit 89 % d'un liquide pur de $p^teb = 79/81$ °C sous 20 mm Hg.

b) L-[(benzyloxycarbonyloxy-5 indol-3 yl)-3 benzyloxycarbonylamino-2 propionyloxy]-2 acétate de (diméthyl-3,3) butyl-2

On agite pendant 20 heures à température ordinaire le mélange suivant :

— 50 g (0,1 mole) d'acide L-(benzyloxycarbonyloxy-5 indol-3 yl)-3 benzyloxycarbonylamino-2 propionique,

— 20 ml (0,1 mole) de dicyclohexylamine,

— 1 g d'iodure de sodium,

— 17,75 g (0,1 mole) de chloracétate de (diméthyl-3,3) butyl-2,

— 300 ml de diméthylformamide,

puis traite suivant l'exemple 1-b. Le produit brut est purifié par chromatographie sur silice avec comme éluant un mélange de toluène 9 p., éther 1 p. On obtient 76 % d'une huile pure présentant un seul spot en C.C.M. sur Kieselgel dans divers systèmes entre autres le mélange : chloroforme 70, Ethanol 30, acide formique 2 (Rf = 0,6) et $[\alpha]^{25}_D = -7,28°$ (Ethanol 1 %).

c) L-[(hydroxy-5 indol-3 yl)-3 amino-2 propionyloxy]-2 acétate de (diméthyl-3,3) butyl-2

On hydrogène sous pression atmosphérique 48 g du produit précédent en solution dans 1 litre d'isopropanol en présence de 2,5 g de Palladium à 5 % sur charbon. Quand la théorie d'hydrogène a été fixée, on filtre le catalyseur, évapore à sec, puis reprend à l'acétate d'éthyle qu'on lave à l'eau. Après évaporation du solvant, on recristallise dans le benzène et obtient 14,5 g (53 %) de cristaux blancs de pF 106/7 °C $[\alpha]^{25}_D = +7,48°$ (1 % acétone).

Exemple 15

L-[(hydroxy-5 indol-3 yl)-3 amino-2 propionyloxy]-2 acétate d'adamantyl-1 méthyle

[(I), R = $CH_2$— 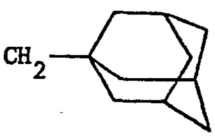 ]

a) L-[(benzyloxycarbonyloxy-5 indol-3 yl)-3 benzyloxycarbonylamino-2 propionyloxy]-2 acétate d'adamantyl-1 méthyle

On agite pendant 20 heures à température ordinaire le mélange suivant :

— 50 g (0,1 mole) d'acide L-(benzyloxycarbonyloxy-5 indol-3 yl)-3 benzyloxycarbonylamino-2 propionique,

— 20 ml de dicyclohexylamine (0,1 mole),

— 24,3 g de chloracétate d'adamantyl-1 méthyle,

— 1 g d'iodure de sodium,

— 500 ml de diméthylformamide,

puis traite selon l'exemple 1-b. L'huile brute résiduelle est purifiée par chromatographie sur silice en éluant par un mélange toluène/éther. On obtient 77 % de produit pur présentant un seul spot par C.C.M. sur gel de silice dans le système habituel $[\alpha]^{25}_D = -8°$ (Ethanol 1 %).

b) L-[(hydroxy-5 indol-3 yl)-3 amino-2 propionyloxy]-2 acétate d'adamantyl-1 méthyle

On hydrogène sous pression atmosphérique 36 g du produit précédent en solution dans 600 ml d'alcool isopropylique en présence de 2,5 g de Palladium à 5 % sur charbon. Quand la théorie d'hydrogène a été fixée, on filtre le catalyseur, évapore le solvant et reprend à l'acétate d'éthyle. Après lavage à l'eau et traitement au noir, on évapore à sec. On recristallise dans le benzène et obtient 68 % de produit fondant à 134 °C et d'$[\alpha]^{25}_D = -0,7°$ (Ethanol 1 %) et $[\alpha]^{25}_D = -26,1°$ (Acétone 1 %).

Exemple 16

L-[(hydroxy-5 indol-3 yl)-3 amino-2 propionyloxy]-2 acétate d'adamantyl-1

[(I), R = 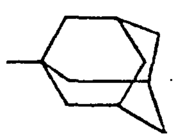 ]

a) Chloracétate d'adamantyl-1

On ajoute lentement à 10 °C et sous azote 24 ml de chlorure de chloracétyle à 30,5 g (0,2 mole)

d'adamantanol-1 et 12 g de magnésic en suspension dans 400 ml de chloroforme puis porte à reflux quelques heures. On refroidit, filtre le minéral et évapore le solvant. Le produit brut est recristallisé dans l'hexane. On obtient 90 % de produit fondant à 79 °C (capillaire).

b) L-[(benzyloxycarbonyloxy-5 indol-3 yl)-3 benzyloxycarbonylamino-2 propionyloxy]-2 acétate d'adamantyl-1

On agite pendant 20 heures à température ordinaire le mélange suivant :
— 41,6 g (0,085 mole) d'acide L-(benzyloxycarbonyloxy-5 indol-3 yl)-3 benzyloxycarbonylamino-2 propionique,
— 20 ml de dicyclohexylamine (0,1 mole),
— 19,5 g de chloracétate d'adamantyl-1,
— 1 g d'iodure de sodium,
— 500 ml de diméthylformamide,
puis traite selon l'exemple 1-b. L'huile résiduelle est purifiée par chromatographie sur silice en éluant par un mélange toluène/éther. On obtient 62 % de produit pur présentant 1 seul spot de Rf 0,6 dans le système habituel et $[\alpha]^{22}_D = -15,85°$ (éthanol 1 %).

c) L-[(hydroxy-5 indol-3 yl)-3 amino-2 propionyloxy]-2 acétate d'adamantyl-1

On hydrogène sous pression atmosphérique 35,5 g (0,052 mole) du produit obtenu au paragraphe 16-b en solution dans 600 ml d'isopropanol et en présence de 4 g de Palladium à 5 % sur charbon. Quand la théorie d'hydrogène a été fixée, on filtre le catalyseur, évapore le solvant et reprend à l'acétate d'éthyle. Après lavage à l'eau. On évapore à sec puis recristallise dans un mélange benzène hexane. On obtient 57 % de produit cristallisé fondant à 90 °C (dec.) $\alpha_D = -9,7°$ (0,5 acétone).

Exemple 17

Acide L-[(hydroxy-5 indol-3 yl)-3 amino-2 propionyloxy]-2 acétique : [(I) : R = H]

a) L-[(benzyloxycarbonyloxy-5 indol-3 yl)-3 benzyloxycarbonylamino-2 propionyloxy]-2 acétate de benzyle

On agite pendant 20 heures à température ordinaire :
— 50 g (0,1 mole) d'acide L-(benzyloxycarbonyloxy-5 indol-3 yl)-3 benzyloxycarbonylamino-2 propionique,
— 20 ml de dicyclohexylamine,
— 18,45 g (0,1 mole) de chloracétate de benzyle,
— 1 g d'iodure de sodium,
— 300 ml de diméthylformamide,
puis traite suivant l'exemple 1-b. L'huile brute est purifiée par chromatographie sur silice avec comme éluant un mélange benzène éther. On obtient 66 % de produit présentant un seul spot de Rf = 0,54 en C.C.M. sur gel de silice dans le système solvant habituel $[\alpha]^{25}_D = -14,73°$ (1 % éthanol).

b) Acide L-[(hydroxy-5 indol-3 yl)-3 amino-2 propionyloxy]-2 acétique

On hydrogène sous pression atmosphérique 36 g (0,056 mole) du produit précédent en solution dans 700 ml d'isopropanol et 150 ml de chloroforme en présence de 4 g de Palladium à 5 % sur charbon. Quand l'absorption d'hydrogène est terminée, on évapore sous vide les solvants, reprend à l'eau, ramène à pH 7,0 avec de l'ammoniaque diluée puis extrait à l'acétate d'éthyle.

Après évaporation du solvant, le produit est purifié par lavage à l'eau et à l'acétone. On obtient 33 % de produit pur fondant à 204/205 °C $[\alpha]^{22}_D = +29,6°$ [1 % ; $NH_4OH$, N/10].

Les composés conformes à l'invention présentent des activités pharmacologiques remarquables, en particulier sur le système nerveux central. Le Tableau I ci-dessous résume le dosage biochimique de sérotonine endogène dans le cerveau des rats. Ces dosages ont été réalisés après administration per os d'une dose unique de 10 mg/kg à des rats Wistar mâles adultes.

Tableau I

| Composés des exemples | 6b | 8b | 12b | 13b | 15b | 14c | (L)5 HTP témoin |
|---|---|---|---|---|---|---|---|
| % augmentation de la sérotonine cérébrale | 22 | 20 | 15 | 26 | 17 | 32 | 10 |

Il est indéniable que l'activité des composés conformes à l'invention est nettement supérieure à celle du 5 HTP.

Toxicité

Les toxicités aiguës des dérivés conformes à l'invention chez la souris sont très inférieures à celles du L 5-HTP. Ainsi les $DL_{50}$ de l'ensemble des composés conformes à l'invention sont supérieures à 1 250 mg/kg, alors que la $DL_{50}$ (per os) du L 5-HTP est égale à 700 mg/kg.

Il résulte de la description qui précède que, quels que soient les modes de mise en œuvre ou d'administration, les dérivés de l'acide [(indol-3 yl)-3 amino-2 propionyloxy]-2 acétique présentent par rapport au 5-HTP précédemment connu, de nombreux avantages et notamment celui d'une activité plus importante, due principalement à leur résistance à la décarboxylase périphérique.

**Revendications**

1. Dérivés de l'acide [(indol-3 yl)-3 amino-2 propionyloxy]-2 acétique caractérisés en ce qu'ils répondent à la formule générale I ci-après :

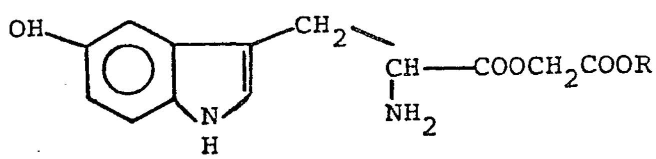

(I)

dans laquelle R représente :

— un atome d'hydrogène,

— un radical alkyle linéaire ou ramifié renfermant 1 à 12 atomes de carbone,

— ou un radical alicyclique mono- ou polycyclique renfermant 5 à 16 atomes de carbone, éventuellement lié par un radical méthylène

ainsi que leurs sels d'addition pharmaceutiquement acceptables.

2. Dérivés selon la Revendication 1 répondant à la formule I, caractérisés en ce qu'ils sont de configuration DL.

3. Dérivés selon la Revendication 1 répondant à la formule I, caractérisés en ce qu'ils sont de configuration L.

4. Composé selon la Revendication 2, caractérisé en ce qu'il est choisi dans le groupe constitué par :

— DL [(hydroxy-5 indol-3 yl)-3 amino-2 propionyloxy]-2 acétate d'éthyle

— DL [(hydroxy-5 indol-3 yl)-3 amino-2 propionyloxy]-2 acétate de butyle

— DL [(hydroxy-5 indol-3 yl)-3 amino-2 propionyloxy]-2 acétate d'octyle

— DL [(hydroxy-5 indol-3 yl)-3 amino-2 propionyloxy]-2 acétate de dodécyle.

5. Composé selon la Revendication 3, caractérisé en ce qu'il est choisi parmi le groupe constitué par :

— L [(hydroxy-5 indol-3 yl)-3 amino-2 propionyloxy]-2 acétate de méthyle

— L [(hydroxy-5 indol-3 yl)-3 amino-2 propionyloxy]-2 acétate d'éthyle

— L [(hydroxy-5 indol-3 yl)-3 amino-2 propionyloxy]-2 acétate de n-propyle

— L [(hydroxy-5 indol-3 yl)-3 amino-2 propionyloxy]-2 acétate de n-butyle

— L [(hydroxy-5 indol-3 yl)-3 amino-2 propionyloxy]-2 acétate de n-pentyle

— L [(hydroxy-5 indol-3 yl)-3 amino-2 propionyloxy]-2 acétate de n-hexyle

— L [(hydroxy-5 indol-3 yl)-3 amino-2 propionyloxy]-2 acétate de n-heptyle

— L [(hydroxy-5 indol-3 yl)-3 amino-2 propionyloxy]-2 acétate de n-octyle

— L [(hydroxy-5 indol-3 yl)-3 amino-2 propionyloxy]-2 acétate de n-dodécyle

— L [(hydroxy-5 indol-3 yl)-3 amino-2 propionyloxy]-2 acétate de diméthyl-3,3 butyl-2

— L [(hydroxy-5 indol-3 yl)-3 amino-2 propionyloxy]-2 acétate d'adamantyl-1 méthyle

— L [(hydroxy-5 indol-3 yl)-3 amino-2 propionyloxy]-2 acétate d'adamantyl-1

Acide L-[(hydroxy-5 indol-3 yl)-3 amino-2 propionyloxy]-2 acétique

6. Médicaments constitués par ou contenant au moins un composé selon l'une quelconque des Revendications 1 à 5.

7. Procédé de préparation des composés selon l'une quelconque des Revendications 1 à 5, caractérisé en ce que l'on bloque la fonction phénol et/ou la fonction amine par un radical $R_2OCO—$ où $R_2$ représente un groupe aliphatique ou aromatique destiné à protéger la fonction phénol et/ou amine, en ce que l'on estérifie l'acide de départ par un composé répondant à la formule

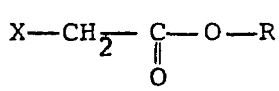

dans laquelle :

X représente un atome d'halogène et de préférence un atome de chlore ou de brome, et

R a la même signification que ci-dessus,

et en ce que l'on débloque ensuite la fonction amine et/ou la fonction phénol.

8. Procédé selon la Revendication 7, caractérisé en ce que les fonctions amine et/ou phénol sont bloquées à l'aide de réactifs pris dans le groupe qui comprend l'

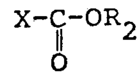

et

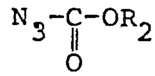

$R_2$ et X ayant la même signification que précédemment.

9. Procédé selon la Revendication 7, caractérisé en ce que la condensation des dérivés de l'acide (hydroxy-5 indol-3 yl)-3 aminopropionique avec l'agent de blocage

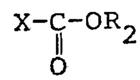

s'effectue en présence d'une base acceptrice d'acide halohydrique et dans un solvant aprotique tel que diméthylformamide ou analogue.

10. Procédé selon la Revendication 7, caractérisé en ce que le déblocage des fonctions $NH_2$ et/ou OH est réalisé par un traitement acide.

11. Procédé selon la Revendication 7, caractérisé en ce que le déblocage des fonctions $NH_2$ et/ou OH est réalisé par hydrogénation catalytique.

**Claims**

1. 2-[3-(Indol-3-yl)-2-aminopropionyloxy] acetic acid derivatives, characterized in that they correspond to the general formula I below :

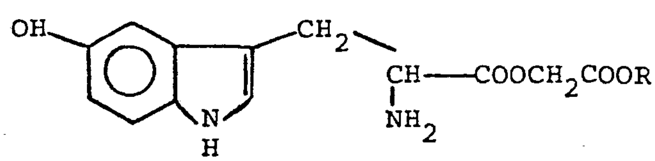

(I)

in which R represents :

— a hydrogen atom,

— a linear or branched alkyl radical containing 1 to 12 carbon atoms, or

— a monocyclic or polycyclic alicyclic radical containing 5 to 16 carbon atoms, which is optionally joined via a methylene radical,

and also their pharmaceutically acceptable addition salts.

2. Derivatives according to Claim 1 corresponding to the formula I, characterized in that they have the DL configuration.

3. Derivatives according to Claim 1 corresponding to the formula I, characterized in that they have the L configuration.

4. A compound according to Claim 2, characterized in that it is chosen from the group comprising :

— ethyl DL-2-[3-(5-hydroxyindol-3-yl)-2-aminopropionyloxy]-acetate,

— butyl DL-2-[3-(5-hydroxyindol-3-yl)-2-aminopropionyloxy]-acetate,

— octyl DL-2-[3-(5-hydroxyindol-3-yl)-2-aminopropionyloxy]-acetate and

— dodecyl DL-2-[3-(5-hydroxyindol-3-yl)-2-aminopropionyloxy]-acetate.

5. A compound according to Claim 3, characterized in that it is chosen from the group comprising :

— methyl L-2-[3-(5-hydroxyindol-3-yl)-2-aminopropionyloxy]-acetate,

— ethyl L-2-[3-(5-hydroxyindol-3-yl)-2-aminopropionyloxy]-acetate,

— n-propyl L-2-[3-(5-hydroxyindol-3-yl)-2-aminopropionyloxy]-acetate,

— n-butyl L-2-[3-(5-hydroxyindol-3-yl)-2-aminopropionyloxy]-acetate,

— n-pentyl L-2-[3-(5-hydroxyindol-3-yl)-2-aminopropionyloxy]-acetate,

— n-hexyl L-2-[3-(5-hydroxyindol-3-yl)-2-aminopropionyloxy]-acetate,

— n-heptyl L-2-[3-(5-hydroxyindol-3-yl)-2-aminopropionyloxy]-acetate,

— n-octyl L-2-[3-(5-hydroxyindol-3-yl)-2-aminopropionyloxy]-acetate,

— n-dodecyl L-2-[3-(5-hydroxyindol-3-yl)-2-aminopropionyloxy]-acetate,

— 3,3 dimethylbut-2-yl L-2-[3-(5-hydroxyindol-3-yl)-2-aminopropionyloxy]-acetate,
— adamantan-1-ylmethyl L-2-[3-(5-hydroxyindol-3-yl)-2-aminopropionyloxy]-acetate,
— adamantan-1-yl L-2-[3-(5-hydroxyindol-3-yl)-2-aminopropionyloxy]-acetate and
— L-2-[3-(5-hydroxyindol-3-yl)-2-aminopropionyloxy]-acetic acid.

6. Medicaments consisting of or containing at least one compound according to any one of Claims 1 to 5.

7. A process for the preparation of the compounds according to any one of Claims 1 to 5, characterized in that the phenol group and/or the amine group is blocked with a radical $R_2OCO$—, in which $R_2$ represents an aliphatic or aromatic group for protecting the phenol and/or amine group, in that the starting acid is esterified with a compound corresponding to the formula

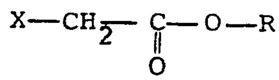

in which :

X represents a halogen atom and preferably a chlorine or bromine atom, and
R has the same meaning as above,

and in that the amine group and/or the phenol group is then unblocked.

8. The process according to Claim 7, characterized in that the amine and/or phenol groups are blocked with reagents taken from the group comprising

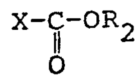

and

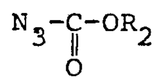

$R_2$ and X having the same meanings as above.

9. The process according to Claim 7, characterized in that the condensation of the 3-(5-hydroxyindol-3-yl)aminopropionic acid derivatives with the blocking agent

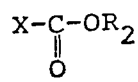

is carried out in the presence of a base which accepts a hydrohalic acid, and in an aprotic solvent such as dimethylformamide or the like.

10. The process according to Claim 7, characterized in that the unblocking of the $NH_2$ and/or OH groups is carried out by an acid treatment.

11. The process according to Claim 7, characterized in that the unblocking of the $NH_2$ and/or OH groups is carried out by catalytic hydrogenation.

**Ansprüche**

1. Derivate von Indol-3-yl-(3-amino-2-propionyloxy)-2-essigsäure, dadurch gekennzeichnet, daß sie der folgenden allgemeinen Formel (I) entsprechen,

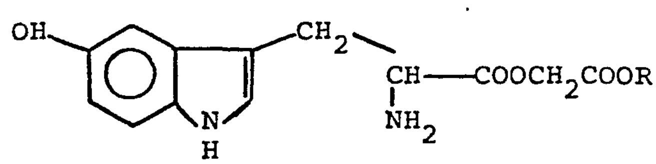

(I)

in der R

— ein Wasserstoffatom,
— einen linearen oder verzweigten Alkylrest, umfassend 1 bis 12 Kohlenstoffatome,
— oder einen alicyclischen, mono- oder polycyclischen Rest, umfassend 5 bis 16 Kohlenstoffatome, gegebenenfalls verbunden durch einen Methylenrest,

darstellt, ebenso wie ihre pharmazeutisch akzeptablen Additionssalze.

2. Derivate gemäß Anspruch 1, entsprechend der Formel (I), dadurch gekennzeichnet, daß sie DL-Konfiguration haben.

3. Derivate gemäß Anspruch 1, entsprechend der Formel (I), dadurch gekennzeichnet, daß sie L-Konfiguration haben.

4. Verbindung gemäß Anspruch 2, dadurch gekennzeichnet, daß sie in der durch

— DL [(5-Hydroxy-Indol-3-yl)-3-amino-2-propionyloxy]-2-essigsäure-ethylester,
— DL [(5-Hydroxy-Indol-3-yl)-3-amino-2-propionyloxy-2-essigsäure-butylester,
— DL [(5-Hydroxy-Indol-3-yl)-3-amino-2-propionyloxy-2-essigsäure-octylester,
— DL [(5-Hydroxy-Indol-3-yl)-3-amino-2-propionyloxy-2-essigsäure-dodecylester

dargestellten Gruppen gewählt wird.

5. Verbindung gemäß Anspruch 3, dadurch gekennzeichnet, daß sie unter der durch

— L [(5-Hydroxy-Indol-3-yl)-3-amino-2-propionyloxy]-2-essigsäuremethylester,
— L [(5-Hydroxy-Indol-3-yl)-3-amino-2-propionyloxy]-2-essigsäureethylester,
— L [(5-Hydroxy-Indol-3-yl)-3-amino-2-propionyloxy]-2-essigsäurepropylester,
— L [(5-Hydroxy-Indol-3-yl)-3-amino-2-propionyloxy]-2-essigsäurebutylester,
— L [(5-Hydroxy-Indol-3-yl)-3-amino-2-propionyloxy]-2-essigsäurepentylester,
— L [(5-Hydroxy-Indol-3-yl)-3-amino-2-propionyloxy]-2-essigsäurehexylester,
— L [(5-Hydroxy-Indol-3-yl)-3-amino-2-propionyloxy]-2-essigsäureheptylester,
— L [(5-Hydroxy-Indol-3-yl)-3-amino-2-propionyloxy]-2-essigsäureoctylester,
— L [(5-Hydroxy-Indol-3-yl)-3-amino-2-propionyloxy]-2-essigsäuredodecylester,
— L [(5-Hydroxy-Indol-3-yl)-3-amino-2-propionyloxy]-2-essigsäure-(3,3-dimethyl-2-butyl)-ester,
— L [(5-Hydroxy-Indol-3-yl)-3-amino-2-propionyloxy]-2-essigsäure-(1-Methyl-adamanthyl)-ester,
— L [(5-Hydroxy-Indol-3-yl)-3-amino-2-propionyloxy]-2-essigsäure

dargestellten Gruppe gewählt wird.

6. Medikamente bestehend aus oder wenigstens eine der Verbindungen gemäß Ansprüchen 1 bis 5 enthaltend.

7. Verfahren zur Herstellung von Verbindungen gemäß einem der Ansprüche 1 bis 5, dadurch gekennzeichnet, daß man die Phenolfunktion und/oder Aminfunktion durch einen Rest $R_2OCO$— blockiert, wo $R_2$ eine aliphatische oder aromatische Gruppe darstellt, die bestimmt ist, die Phenol- und/oder Amin-Funktion zu schützen, wobei man die Ausgangssäure verestert durch eine Verbindung entsprechend der Formel

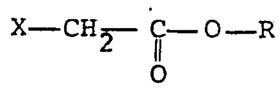

in der

X ein Halogenatom und vorzugsweise ein Chlor- oder Bromatom darstellt, und R dieselbe Bedeutung wie oben hat, und wobei man dann die Amin- und/oder Phenol-Funktion deblockiert.

8. Verfahren gemäß Anspruch 7, dadurch gekennzeichnet, daß die Amin- und/oder Phenol-Funktion blockiert sind mit Hilfe vom Reagenzien, genommen in der Gruppe die

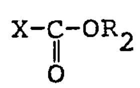

und

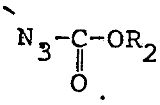

umfaßt, wobei $R_2$ und X dieselbe Bedeutung haben wie vorangehend.

9. Verfahren gemäß Anspruch 7, dadurch gekennzeichnet, daß die Kondensation von Derivaten von (5-Hydroxy-Indol-3-yl)-3-amino-propionsäure mit dem Blockierungsagens

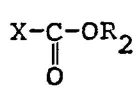

durchgeführt wird in Anwesenheit einer Akzeptierungsbase von Halogenwasserstoffsäure und in einem aprotischen Lösungsmittel wie Dimethylformamid oder analogem.

10. Verfahren gemäß Anspruch 7, dadurch gekennzeichnet, daß die Deblockierung der $NH_2$- und/oder OH- Funktionen durch eine Säurebehandlung durchgeführt wird.

11. Verfahren gemäß Anspruch 7, dadurch gekennzeichnet, daß die Deblockierung der $NH_2$- und/oder OH- Funktionen durch katalytische Hydrierung durchgeführt wird.